(19) Europäisches Patentamt
European Patent Office
Office européen des brevets

(11) **EP 4 371 557 A1**

(12) **EUROPEAN PATENT APPLICATION**
published in accordance with Art. 153(4) EPC

(43) Date of publication:
**22.05.2024 Bulletin 2024/21**

(21) Application number: 22842373.7

(22) Date of filing: **07.07.2022**

(51) International Patent Classification (IPC):
**A61K 9/127** (2006.01) **A61K 47/69** (2017.01)
**A61K 33/32** (2006.01) **A61K 45/06** (2006.01)
**A61P 35/00** (2006.01)

(86) International application number:
**PCT/KR2022/009876**

(87) International publication number:
**WO 2023/287111 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.07.2021 KR 20210091866**

(71) Applicant: **Dr. Cure Co., Ltd.**
**Gwangju 61003 (KR)**

(72) Inventors:
• **PARK, In Kyu**
**Gwangju 61698 (KR)**
• **JEONG, Yong Yeon**
**Gwangju 61704 (KR)**

(74) Representative: **Stolmár & Partner**
**Patentanwälte PartG mbB**
**Blumenstraße 17**
**80331 München (DE)**

(54) **MICELLE COMPLEX AND DRUG CARRIER COMPRISING SAME**

(57) The present invention relates to a micelle complex and a drug carrier. By supporting hydrophobic manganese oxide particles in the micelles, the complex is stably protected *in vivo* and easily delivered to a tumor site. Further, the micelles have a hydrophilic portion and a hydrophobic portion bound by a reactive oxygen species (ROS) cleavable linker in order to be degraded in response to a reactive oxygen species of a target site, and thus can efficiently release the hydrophobic manganese oxide particles. The released hydrophobic manganese oxide particles generate oxygen and changes the microenvironment of the target site, and thus may enhance the therapeutic effect of a drug carried on the micelles.

[FIG. 10]

EP 4 371 557 A1

**Description**

[Technical Field]

The present invention relates to a complex and a drug delivery system.
The present invention relates to a composition for enhancing radiation therapy.
The present invention relates to a composition for enhancing photodynamic therapy.
The present invention relates to a composition for enhancing ultrasound dynamic therapy.

[Background Art]

A drug delivery system refers to a medical technique that can efficiently deliver a required amount of drugs, such as proteins, nucleic acids or other small molecules by minimizing side effects while maximizing efficacy and effects of existing drugs. This technique capable of saving costs and time required for development of new drugs has recently become one of advanced techniques that create a new added value in the pharmaceutical industry, in combination with nanotechnique. Since the late 1980s, in particular, companies in the technically advanced countries such as United States, Japan, etc. have made every effort to develop drug delivery systems along with the development of new drugs.
Tumor hypoxia is an ex *vivo* oxygen stress around most solid tumors due to a distorted tumor vasculature surrounding hyper-proliferated cancer cells. Studies have shown that hypoxic environments not only confer multidrug resistance to chemotherapy in cancer cells due to poor penetration of chemotherapeutic drugs in avascular hypoxic solid tumors, but also promote secretion of multidrug-resistant P-GP1 (permeability glycoproteins) transporter proteins in cancer cells. Further, an oxygen level depleted by hypoxic cancer cells has been found to reduce radio-sensitivity and efficacy of radiation-based treatment methods by up to 3-fold.
Therefore, there is a need for research on agents that can be efficiently delivered to hypoxic sites, effectively suppress the hypoxic environment and change the microenvironment of the target tissue to maximize the effect of the drug.

[Summary of Invention]

[Problems to be Solved by Invention]

An object of the present invention is to provide a complex in which manganese oxide particles whose surfaces are modified with hydrophobic substituents are supported on micelles and can circulate *in vivo* while maintaining stability.
Another object of the present invention is to provide a drug delivery system in which hydrophobic manganese oxide particles react with active oxygen at a tumor site to induce oxygen generation so as to change the microenvironment of a target tissue, thereby improving the effect of a drug carried on micelles.
In addition, another object of the present invention is to provide a composition for enhancing radiation therapy.
Further, another object of the present invention is to provide a composition for enhancing photodynamic therapy.
Furthermore, another object of the present invention is to provide a composition for enhancing ultrasound dynamic therapy.

[Means for Solving Problems]

1. A complex including manganese oxide particles having a hydrophobic substituent on the surface thereof, as well as micelles carrying the same, wherein the micelles have a hydrophilic portion and a hydrophobic portion bound by a reactive oxygen species (ROS) cleavable linker.
2. The complex according to the above 1, the hydrophobic substituent is a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a halogen group, a C1 to C30 ester group or a halogen-containing group.
3. The complex according to the above 1, wherein the manganese oxide is any one of MnO, $Mn_2O_3$, $MnO_2$, $Mn_3O_4$ and $Mn_2O_5$.
4. The complex according to the above 1, wherein the manganese oxide particles have a particle diameter of 10 to 30 nm.
5. The complex according to the above 1, wherein the linker is a thioketal linker, TSPBA linker ($N^1$-(4-boronoben-zyl)-$N^3$-(4-boronophenyl)-$N^1$,$N^1$,$N^3$,$N^3$-tetramethylpropane-1,3-diaminium) or AA linker (aminoacrylate).
6. The complex according to the above 1, wherein the hydrophilic portion of the micelle includes a water-soluble polymer, the hydrophobic portion includes a hydrophobic hydrocarbon compound, and the water-soluble polymer and the hydrophobic hydrocarbon compound are bound by a thioketal linker.

7. The complex according to the above 6, wherein the water-soluble polymer is polyethylene glycol, acrylic acid polymer, methacrylic acid polymer, polyamidoamine, poly(2-hydroxypropyl methacrylamide) polymer, water-soluble polypeptide, water-soluble polysaccharide or polyglycerol.

8. The complex according to the above 6, wherein the hydrophobic hydrocarbon compound is hexadecylamine, heptadecylamine, stearamine, nonadecylamine, dodecylamine, N,N-bis(2-hydroxyethyl)laurylamine, linoleic acid, linolenic acid, palmitic acid, oleylamine, hexadecanoic acid, stearic acid, oleic acid, eicosencic acid or erucic acid.

9. The complex according to the above 1, wherein the complex includes 5 to 10 parts by weight of the manganese oxide particles based on 100 parts by weight of the micelles.

10. The complex according to the above 1, wherein the micelles have a particle diameter of 150 to 250 nm.

11. A drug delivery system, including manganese oxide particles having a hydrophobic substituent on the surface thereof and micelles carried with a drug, wherein the micelles have a hydrophilic portion and a hydrophobic portion bound by a ROS-cleavable linker.

12. A composition for enhancing radiation therapy, including the complex according to any one of the above 1 to 10 or the drug delivery system according to the above 11.

13. A composition for enhancing photodynamic therapy, including the complex according to any one of the above 1 to 10 or the drug delivery system according to the above 11.

14. A composition for enhancing ultrasound dynamic therapy, including the complex according to any one of the above 1 to 10 or the drug delivery system according to the above 11.

[Advantageous effects]

The present invention relates to a complex in which hydrophobically surface-modified manganese oxide particles are supported on micelles. By supporting or carrying hydrophobic manganese oxide in the micelles, the complex is stably protected *in vivo* and easily delivered to a target site.

In addition, the a hydrophilic portion and a hydrophobic portion of the micelles are bound by a ROS-cleavable linker and decomposed in response to active oxygen at a tumor site, thereby efficiently releasing hydrophobic manganese oxide particles. Further, the released hydrophobic manganese oxide generates oxygen to change the microenvironment of the target site, thereby improving the effect of the drug carried on the micelles.

[Brief Description of Drawings]

FIG. 1 illustrates a manufacturing process of hydrophobic manganese oxide nanoparticles.

FIG. 2 illustrates a reaction scheme for preparing PTS of Preparation Example 2.

FIG. 3 illustrates XPS spectrum analysis results of hydrophobic manganese oxide having a C12 alkyl group of Preparation Example 1.

FIG. 4 illustrates EDS elemental analysis results of hydrophobic manganese oxide having a C12 alkyl group of Preparation Example 1.

FIG. 5 illustrates TEM analysis results of hydrophobic manganese oxide having a C12 alkyl group of Preparation Example 1.

FIG. 6 is a bright field image of cancer cells used in Experimental Example 2-1, indicating that all cancer cells used in the experiment are stable and healthy.

FIG. 7 illustrates results of confirming oxygen generation effects of the hydrophobic manganese oxide-micelle complex of Experimental Example 2-1 as a green fluorescent protein (GFP) image.

FIG. 8 illustrates results of confirming oxygen generating effects of the hydrophobic manganese oxide-micelle complex of Experimental Example 2-2 by FACS (Fluorescence-activated cell sorting) and fluorescence microscopy.

FIG. 9 is an experimental schematic view of Experimental Example 3.

FIG. 10 illustrates effects of reducing the tumor volume and body weight change of the hydrophobic manganese oxide-micelle complex in Experimental Example 3.

FIG. 11 illustrates results of visually confirming the effects of reducing the tumor volume of the hydrophobic manganese oxide-micelle complex in Experimental Example 3.

FIG. 12 illustrates results of confirming effects of increasing the survival rate of the hydrophobic manganese oxide-micelle complex in Experimental Example 3 on a mouse model of hepatocellular carcinoma.

FIG. 13 illustrates results of confirming tumor necrosis effects of the hydrophobic manganese oxide-micelle complex on hepatocellular carcinoma cells in Experimental Example 3.

FIG. 14 illustrates results of confirming apoptotic effects of the hydrophobic manganese oxide-micelle complex on hepatocellular carcinoma cells in Experimental Example 3.

FIG. 15 illustrates a process for preparing hydrophobic manganese oxide according to the length of an alkyl group of Preparation Example 1.

FIG. 16 is a TEM image of hydrophobic manganese oxide of Preparation Example 1.

[Mode for Carrying out Invention]

The present invention provides a complex, which includes manganese oxide particles having a hydrophobic substituent on the surface thereof and micelles carrying the same, wherein the micelles have a hydrophilic portion and a hydrophobic portion bound by a reactive oxygen species (ROS) cleavable linker.

The complex may include micelles formed of amphiphilic molecules in which a hydrophilic portion and a hydrophobic portion are bound by a reactive oxygen species (ROS) cleavable linker; and manganese oxide particles which are supported on cores of the micelles and hydrophobically modified by binding a hydrophobic substituent to the surface thereof.

The present invention may include manganese oxide particles having a hydrophobic substituent on the surface thereof (hereinafter, referred to as hydrophobic manganese oxide).

The hydrophobic manganese oxide particles are hydrophobically modified by treating manganese oxide to have a hydrophobic substituent on the surface thereof, so that the manganese oxide can be stably supported on the hydrophobic portion of the micelle.

The hydrophobic substituent may be, for example, a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a halogen group, a C1 to C30 ester group, or a halogen-containing group, and specifically, a substituted or unsubstituted C1 to C30 alkyl group.

In one embodiment of the present invention, the manganese oxide may be treated with, for example, 1-dodecanethiol, 1-octadecanethiol or 1-hexanediol to have a hydrophobic substituent on the surface thereof.

The manganese oxide may be any one of $MnO$, $Mn_2O_3$, $MnO_2$, $Mn_3O_4$ and $Mn_2O_3$, and preferably $Mn_3O_4$, but it is not limited thereto.

In one embodiment of the present invention, the manganese oxide may be $Mn_3O_4$ synthesized by reducing manganese chloride with an alkaline solution (e.g., NaOH) to generate $Mn(OH)_2$ and to be decomposed into MnO, followed by oxidizing the product with oxygen in the air.

As described above, the surface of the manganese oxide particles may be modified with a hydrophobic substituent to be stably supported on cores of the micelles.

The hydrophobic manganese oxide particles may have a particle diameter of 10 to 30 nm. When the particle diameter of the hydrophobic manganese oxide particles is within the above range, the particles may be evenly distributed and stably supported on the hydrophobic cores of the micelles, and the reactivity with active oxygen at the target site may be increased thus to increase oxygen generation.

Micelles are formed of amphiphilic molecules including a hydrophilic portion and a hydrophobic portion. The hydrophilic portion and the hydrophobic portion are bound by a ROS-cleavable linker, and the linker is a linking group for connecting the hydrophilic portion and the hydrophobic portion, has high sensitivity to active oxygen and reacts with the active oxygen, thereby resulting in breakage of the bond.

The linker may be, for example, a thioketal linker, a TSPBA linker ($N^1$-(4-boronobenzyl)-$N^3$-(4-boronophenyl)-$N^1$,$N^1$,$N^3$,$N^3$-tetramethylpropane-1,3-diaminium) or AA linker (aminoacrylate), etc., but it is not limited thereto.

The hydrophilic portion of the amphiphilic molecule may include a water-soluble polymer. The water-soluble polymer may have a weight average molecular weight in the range of 500 to 10,000 g/mol, preferably 800 to 5,000 g/mol, and more preferably 1,000 to 3,000 g/mol. The water-soluble polymer may be polyethylene glycol, acrylic acid polymer, methacrylic acid polymer, polyamidoamine, poly(2-hydroxypropyl methacrylamide) polymer, water-soluble polypeptide, water-soluble polysaccharide or polyglycerol, or derivatives thereof, etc., but it is not limited thereto.

The water-soluble polypeptide may be specifically selected from polyglutamic acid, polyaspartic acid, polyaspartamide, and copolymers thereof, but it is not limited thereto.

The water-soluble polysaccharides may be specifically selected from hyaluronic acid, alginic acid, dextran, pectin, chondroitin sulfate, heparin, hydroxypropyl cellulose, hydroxyethyl cellulose, methylcellulose, sodium carboxymethylcellulose, hydroxypropyl methylcellulose acetate succinate and derivatives thereof, but it is not limited thereto.

The hydrophobic portion of the amphiphilic molecule may include a C12 to C22 aliphatic hydrocarbon group. The aliphatic hydrocarbon group may be a C12 to C22 alkyl group or a C12 to C22 alkenyl group, and specifically may be a C12 to C22 alkyl group.

In one embodiment, the C12 to C22 alkyl group may be an octadecyl group.

In the micelles, the hydrophilic portion may consist of a water-soluble polymer, while the hydrophobic portion may consist of a hydrophobic hydrocarbon compound.

The micelles may be formed of amphiphilic molecules in which a water-soluble polymer and a hydrophobic hydrocarbon compound are bound by a ROS-cleavable linker. The micelles may be made of an amphiphilic polymer compound in which a water-soluble polymer and a hydrophobic hydrocarbon compound are covalently bonded with a thioketal linker (TL), respectively. Specifically, one end of the water-soluble polymer and one end of the hydrophobic hydrocarbon compound may be covalently bonded with a thioketal linker through an amide group, respectively, and then self-assembled in an aqueous phase, thereby producing the micelles.

The thioketal linker is a linking group for connecting a water-soluble polymer and a hydrophobic hydrocarbon compound, which has very high sensitivity to active oxygen and excellent bonding stability under normal physiological conditions, thus to prevent the micelles from early leaking hydrophobic manganese oxide particles during circulation in the blood.

The hydrophobic hydrocarbon compound is a compound including a hydrocarbon group having hydrophobic properties, and the hydrocarbon group may have an aliphatic hydrocarbon group having 12 to 22 carbon atoms, wherein the hydrocarbon group may have an aliphatic hydrocarbon group, specifically a straight-chain aliphatic hydrocarbon group.

The hydrophobic hydrocarbon compound may include hexadecylamine, heptadecylamine, stearamine, nonadecylamine, dodecylamine, N,N-bis(2-hydroxyethyl)laurylamine), linoleic acid, linolenic acid, palmitic acid, oleylamine, hexadecanoic acid, stearic acid, oleic acid, eicosencic acid, or erucic acid, etc., but it is not limited thereto.

The hydrophobic hydrocarbon compound may have a weight average molecular weight of 100 to 500 g/mol, preferably 150 to 350 g/mol, and more preferably 180 to 300 g/mol, but it is not limited thereto.

If the hydrophobic portion of the amphiphilic molecule includes a hydrophobic hydrocarbon compound (e.g., a C12 to C22 aliphatic hydrocarbon group), the hydrophobic hydrocarbon compound forms a hydrophobic core, such that a hydrophobic core portion of the micelles may have a predetermined degree of fluidity. The fluidity has an advantage over the hydrophobic core portion formed by a hydrophobic polymer having low fluidity in an aspect that it can rapidly release hydrophobic manganese oxide placed in the hydrophobic core in response to active oxygen. A long-chain aliphatic hydrocarbon group forms a hydrophobic core to impart sufficient hydrophobicity to the hydrophobic core, thereby stably supporting hydrophobic manganese oxide while providing a predetermined degree of fluidity, such that a thioketal linker with ROS-labile property to active oxygen becomes sensitive to and reacts with the active oxygen around target tissues, and thus, can be easily decomposed.

Further, compared to the case where a large hydrophobic polymer is used, even if the micelles formed by self-assembly in an aqueous phase has a relatively small size, a sufficient amount of hydrophobic manganese oxide may be stably collected in the hydrophobic core due to a certain degree of fluidity of the aliphatic hydrocarbon group.

A ratio of the weight average molecular weights between the water-soluble polymer and the hydrophobic hydrocarbon compound may satisfy Equation 1 below.

$$[\text{Equation 1}]$$

$$0.01 < HC/WSP < 0.3$$

(In Equation 1, WSP means the weight average molecular weight of a water-soluble polymer, and HC means the molecular weight of a hydrophobic hydrocarbon compound).

In Equation 1, a value of HC/WSP may be 0.01 to 0.3, specifically 0.04 to 0.25, and more specifically 0.08 to 0.2. Unlike conventional micelles, the micelles formed of water-soluble polymers and hydrophobic hydrocarbon compounds, which satisfy Equation 1 above, have a smaller molecular weight of hydrophobic hydrocarbon groups and thus exhibit excellent self-assembly in the aqueous phase including a hydrophobic material, and are thermodynamically controlled rather than kinetically, thereby attaining an advantage of excellent reproducibility. Moreover, the resulting micelles have a small molecular weight in a hydrophobic region, such that these micelles preferably do not aggregate into secondary particles, have better dispersibility and stability, have a small particle size, and may exhibit significantly improved colloidal stability.

The complex of the present invention may include 5 to 10 parts by weight ("wt. parts") of manganese oxide particles based on 100 wt. parts of the micelles. When the hydrophobic manganese oxide is included in the micelles within the above range, the micelles may stably capture the hydrophobic manganese oxide, and the hydrophobic manganese oxide may be effectively released in the tumor site. Further, the hydrophobic manganese oxide is included in a desirable amount to increase the amount of oxygen generated by the hydrophobic manganese oxide released in the target site, thereby changing the microenvironment of the target site to improve therapeutic effects of the drug.

In the complex of the present invention, the micelles may have a diameter of 150 to 250 nm. When the micelles have a diameter within the above range, circulation *in vivo* is facilitated, and decomposition of a thioketal link by active oxygen and release of hydrophobic manganese oxide may be performed well.

The present invention provides a drug delivery system which includes manganese oxide particles having a hydrophobic substituent on the surface thereof, and micelles carrying a drug, wherein the micelles have a hydrophilic portion and a hydrophobic portion bound by a ROS-cleavable linker.

The drug delivery system may include: micelles formed of amphiphilic molecules in which a hydrophilic portion and a hydrophobic portion are bound by a ROS-cleavable linker; manganese oxide particles which are supported on cores of the micelles and hydrophobically modified by binding a hydrophobic substituent to the surface thereof; and a drug carried on the micelles.

The hydrophilic portion, hydrophobic portion, reactive oxygen-cleavable linker, amphiphilic molecule, hydrophobic substituent, hydrophobic manganese oxide and micelle are the same as described above.

The drug delivery system of the present invention may be a system in which various drugs are carried in micelles. The drug carried in the drug delivery system may be released together with hydrophobic manganese oxide at a hypoxic site of the target site, and may exhibit higher efficacy due to the tissue microenvironment changed by oxygen generation induced from the hydrophobic manganese oxide.

The drug may be a photosensitizer. The type of photosensitizer is not limited as long as it is a material generally used as the photosensitizer, but may include, for example, any one or two or more selected from the group consisting of a porphyrin-based compound, a chlorine-based compound, a bacteriochlorin-based compound, a phthalocyanine-based compound, a naphthalocyanine-based compound, a 5-aminolevulin ester-based compound and the like.

Further, the drug may be, for example, a general medication, a drug, a prodrug or a target group, or a drug or prodrug including the target group. For example, the drug may include: cardiovascular drugs, especially antihypertensives (e.g., calcium channel blockers, or calcium antagonists) and antiarrhythmics; congestive heart failure medication; muscle contraction; vasodilators; ACE inhibitors; diuretic; deoxygenation dehydrogenase inhibitors; cardiac glycosides; phosphodiesterase inhibitors; blockers; β-blockers; sodium channel blockers; potassium channel blockers; β-adrenergic agonists; platelet inhibitors; angiotensin II antagonists; anticoagulants; thrombolytic agents; bleeding medication; anemia treatment; thrombin inhibitor; antiparasitic agents; antibacterial agent; anti-inflammatory agents, particularly non-steroidal anti-inflammatory drugs (NSAIDs), more particularly COX-2 inhibitors; steroidal anti-inflammatory agents; prophylactic anti-inflammatory; antiglaucoma agents; mast cell stabilizers; mydriatics; drugs affecting the respiratory system; allergic rhinitis medicine; alpha-adrenergic antagonists; corticosteroids; chronic obstructive pulmonary disease medication; xanthine-oxidase inhibitors; anti-arthritic agents; gout medication; autopotency drugs and autopotency drug antagonists; antituberculous agents; antifungal agents; antiprotozoal agents; helminthic; antiviral agents, in particular respiratory antiviral agents, antiviral agents against herpes, cytomegalovirus, human immunodeficiency virus and hepatitis infections; therapeutics of leukemia and Kaposi's sarcoma; pain management agents, especially opioids, including anesthetics and analgesics, opioid receptor agonists, opioid receptor partial agonists, opioid antagonists, opioid receptor mixed agonist-antagonists; neuroleptics; sympathomimetics; adrenergic antagonists; drugs that affect neurotransmitter uptake and release; anticholinergics; antihemorrhoidal agents; therapeutics for prevention or treatment of radiation or chemotherapy effects; adipogenic agents; fat reducing agents; anti-obesity agents such as lipase inhibitors; sympathomimetics; gastric ulcer and inflammation treatment agents such as proton pump inhibitors; prostaglandins; VEGF inhibitors; antihyperlipidemic agents, especially statins; drugs affecting the central nervous system (CNS) such as antipsychotic, antiepileptic and antiseizure drugs (anticonvulsants), psychoactive agents, stimulants, anxiolytics and hypnotics; antidepressants; antiparkinsonian agents; hormones and fragments thereof, such as sexual hormones; growth hormone antagonists; gonadotropin releasing hormone and its analogues; steroid hormone and its antagonists; selective estrogen modulators; growth factors; antidiabetic agents such as insulin, insulin fragments, insulin analogues, glucagon-like peptides and hypoglycemic agents; H1, H2, H3 and H4 antihistamines; peptide, protein, polypeptide, nucleic acid and oligonucleotide drugs; analogs, fragments and variants of natural proteins, polypeptides, oligonucleotides and nucleic acids, etc.; medications used to treat migraines; asthma medication; cholinergic antagonists; glucocorticoids; androgens; antiandrogen; inhibitors of adrenocorticoid biosynthesis; osteoporosis agents such as biphosphonates; antithyroid drugs; sunscreens, anti-ultraviolet protectors and filters; cytokine antagonists; antitumor agents; anti-alzheimer's; HMGCoA reductase inhibitors; fibrates; cholesterol absorption inhibitors; HDL cholesterol raising agents; triglyceride reducing agents; antiaging or anti-wrinkle agents; precursor molecules for production of hormones; proteins such as collagen and elastin, antibacterial agents; anti-acne agents; antioxidants; hair treatment and skin lightening agents; sun creams, anti-ultraviolet protectors and filters; variants of human apolipoprotein; precursor molecules for production of hormones; their proteins and peptides; amino acid; plant extracts such as grape seed extract; DHEA; isoflavones; vitamins, nutrients including phytosterols and iridoid glycosides, sesquiterpene lactones, terpenes, phenolic glycosides, triterpenes, hydroquinone derivatives and phenylalkanones; antioxidants such as retinol and other retinoids including retinoic acid and coenzyme Q10; omega-3-fatty acids; glucosamine; nucleic acids, oligonucleotides, antisense drugs; enzyme; coenzyme; cytokine analogs; cytokine agonists; cytokine antagonists; immunoglobulins; antibodies; antibody medicine; gene therapy agents; lipoprotein; erythropoietin; vaccine; small molecule therapeutics for treatment or prevention of human and animal diseases such as allergies/asthma, arthritis, cancer, diabetes, growth disorders, cardiovascular disease, inflammation, immune disorders, baldness, pain, eye diseases, epilepsy, gynecological disorders, CNS diseases, viral infections, bacterial infections, parasitic infections, GI disorders, obesity and blood diseases, and the like, but they are not limited thereto.

Further, the present invention provides a composition for enhancing radiation therapy, which includes the above-described complex or the above-described drug delivery system.

A disease that is a target of radiation therapy is not limited as long as it can exhibit therapeutic effects by irradiation, but may be, for example, cancer.

The cancer may be one selected from the group consisting of hepatocellular carcinoma, liver metastasis cancer, colorectal cancer, lung cancer, breast cancer, biliary tract cancer, gallbladder cancer, pancreatic cancer, cervical cancer, esophageal cancer, brain cancer, rectal cancer, prostate cancer, and head and neck cancer.

The composition may be administered before irradiation, after irradiation, or simultaneously with irradiation. Specifically, the composition may be administered before irradiation.

When administration of the composition of the present invention and irradiation are conducted together, superior effects of radiation therapy may be obtained compared to radiation alone.

The above-described complex effectively releases hydrophobic manganese oxide at hypoxic sites of tumor tissue. The hypoxic sites are difficult to penetrate various drugs and show resistance to radiation, which is a major cause of attenuating the therapeutic effects by drugs or radiation.

The composition of the present invention may effectively release hydrophobic manganese oxide at the hypoxic site of tumor tissue thus to convert active oxygen in the hypoxic site of tumor tissue into oxygen, thereby enhancing effects of radiation therapy.

Further, the present invention provides a composition for enhancing photodynamic therapy, which includes the above-described complex or the above-described drug delivery system.

A drug used for photodynamic therapy may be additionally carried on the complex.

The drug may be a photosensitizer. In general, materials used as a photosensitizer are not limited, but may include, for example, any one or two or more selected from the group consisting of a porphyrin-based compound, a chlorine-based compound, a bacteriochlorin-based compound, a phthalocyanine-based compound, a naphthalocyanine-based compound, a 5-aminolevulin ester-based compound and the like.

A disease that is a target of photodynamic therapy is not limited as long as it can exhibit therapeutic effects by laser irradiation, but may be, for example, cancer.

The cancer may be one selected from the group consisting of hepatocellular carcinoma, liver metastasis cancer, colorectal cancer, lung cancer, breast cancer, biliary tract cancer, gallbladder cancer, pancreatic cancer, cervical cancer, esophageal cancer, brain cancer, rectal cancer, prostate cancer, and head and neck cancer.

The composition may be administered before laser irradiation, after laser irradiation, or simultaneously with laser irradiation. Specifically, the composition may be administered before laser irradiation.

When administration of the composition of the present invention and laser irradiation are conducted together, superior effects of photodynamic therapy may be obtained compared to laser irradiation alone.

The photosensitizer carried in the complex may be released at a hypoxic site of the target site together with the hydrophobic manganese oxide. The released hydrophobic manganese oxide may change the microenvironment of the target site by generating oxygen at the target site, and may enhance therapeutic effects by the photosensitizer.

Further, the present invention provides a composition for enhancing ultrasound dynamic therapy, which includes the above-described complex or the above-described drug delivery system.

A disease that is a target of ultrasound dynamic therapy is not limited as long as it can exhibit therapeutic effects by ultrasound treatment, but may be, for example, cancer.

The cancer may be one selected from the group consisting of hepatocellular carcinoma, liver metastasis cancer, breast cancer, biliary tract cancer, pancreatic cancer, and prostate cancer.

The composition may be administered before ultrasound irradiation, after ultrasound irradiation, or simultaneously with ultrasound irradiation. Specifically, the composition may be administered before ultrasound irradiation.

When administration of the composition of the present invention and ultrasonic irradiation are conducted together, superior effects of ultrasound dynamic therapy may be obtained compared to ultrasonic irradiation alone.

The composition of the present invention may effectively release hydrophobic manganese oxide at the hypoxic site of tumor tissue thus to convert active oxygen in the hypoxic site of tumor tissue into oxygen, thereby enhancing effects of ultrasound dynamic therapy.

Hereinafter, the present invention will be described in more detail by way of the following examples in order to concretely describe the present invention.

## Preparation Example 1. Preparation of hydrophobic manganese oxide

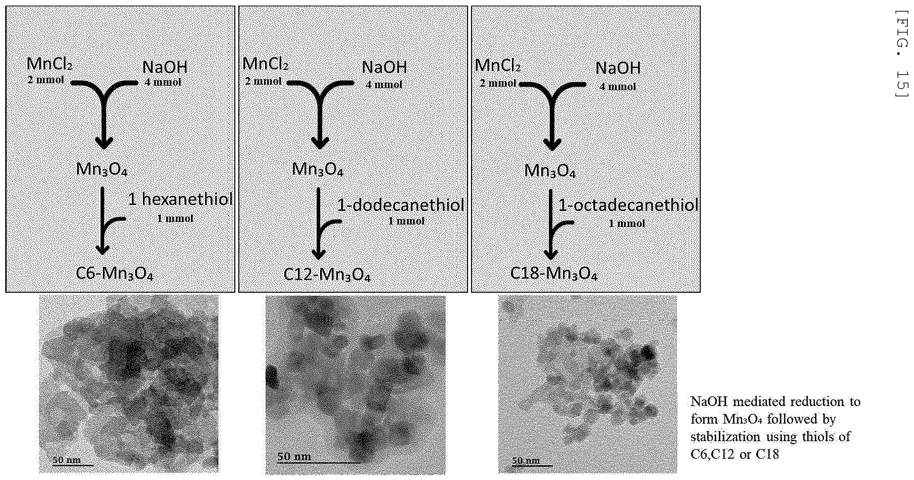

To prepare hydrophobic manganese oxide particles having a C6, C12 or C18 alkyl group, the materials in Table 1 were used, and the hydrophobic manganese oxide production process according to a length of alkyl group is shown in FIG. 15.

[TABLE 1]

| C6 alkyl group | C12 alkyl group | C18 alkyl group |
|---|---|---|
| Manganese (II) chloride dihydrate (MnCl$_2$.2H$_2$O) 2 mmol (323.74 mg in 2.5 distilled water) | | |
| NaOH4 mmol (160 mg in 2.5 ml distilled water) | | |
| 1-hexanethiol 1 mmol (142 μl or 118.17 mg) | 1-dodecanethiol 1 mmol (239 μl or 202.40 mg) | 1-octadecanethiol 1 mmol (286.56 mg in 1ml chloroform) |

After completely dissolving MnCl$_2$, 4 mmol of NaOH was added to 2 mmol of MnCl$_2$ and stirred strongly to obtain Mn(OH)$_2$ through a reductive reaction, then it was confirmed that a sample turned brown. Then, Mn(OH)$_2$ was decomposed into MnO, and MnO was oxidized to Mn$_3$O$_4$ by oxygen in the air. While stirring strongly 4 mmol of Mn$_3$O$_4$ obtained above, it reacted with 1 mmol of 1-hexanethiol, 1-dodecanethiol or 1-octadecanethiol to produce hydrophobic manganese oxide particles having each C6, C12 or C18 alkyl group on the surface of Mn$_3$O$_4$. The formed precipitate was washed with ethanol (5,000 rpm, 5 minutes, 5 times), vacuum dried or air dried, and the dried precipitate was washed twice with distilled water and then lyophilized to finally obtain hydrophobic manganese oxide particles.

**Preparation Example 2. Preparation of micelles**

Step 1: Preparation of thioketal linker (TL)

49 mmol of 3-mercaptopropionic acid was dissolved in 98 mmol of anhydrous acetone, and the resulting product was cooled while stirring constantly at 23 °C for 6 hours until crystallization. The crystallized product was filtered, rinsed with n-hexane, and then rinsed once more with cold distilled water, followed by lyophilization.

Step 2: Synthesis of thioketal linker-conjugated polyethylene glycol (PEG-TL)

254 mg of the thioketal linker (TL) prepared in Step 1 and 200 mg of methoxy-polyethylene glycol amine (PEG-AM) having a molecular weight of 2 kDa were mixed with 10 ml of N,N-dimethylformamide (DMF) and mixed at room temperature to prepare a mixed solution. Thereafter, 278 mg of 1-ethyl-3-(3-dimethylaminopropyl)carbodiimide [EDC] and 345 mg of N-hydroxysuccinimide (NHS) were added to the mixed solution prepared above, and the mixture was stirred for 16 hours. After stirring, the mixed solution was dialyzed under a condition that a molecular cut-off (MWCO) against distilled water is 1,000, so as to remove reaction by-products, followed by lyophilization. The lyophilized powder was re-dissolved in 1 ml of DMF and precipitated in cold diethyl ether 5 times. Then, the resulting product was vacuum dried to prepare thioketal linker-conjugated polyethylene glycol (PEG-TL).

Step 3: Production of polyethylene glycol-TL-stearamine (PTS)

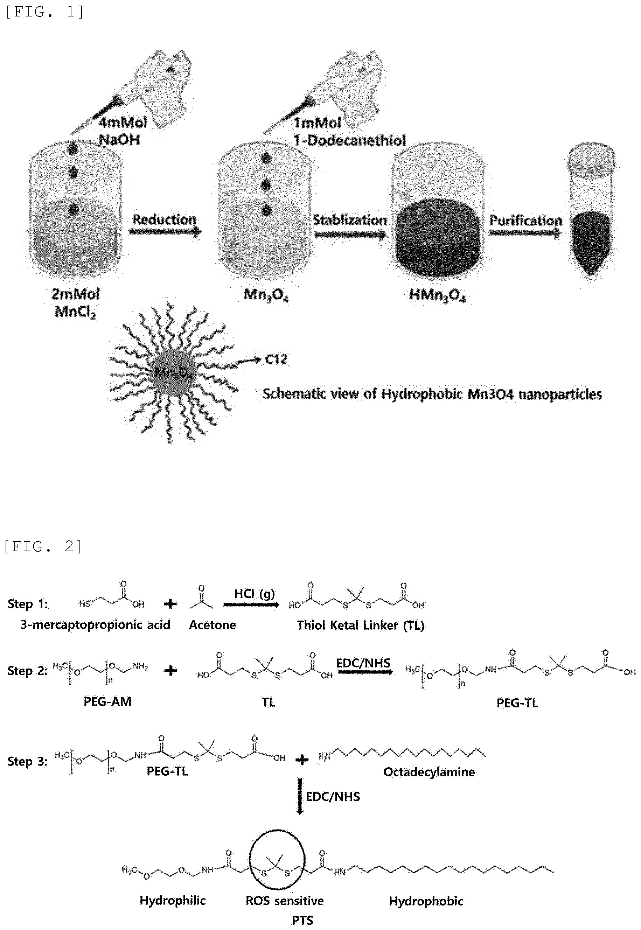

100 mg of PEG-TL prepared in Step 2 above, 80 ml of triethylamine (TEA) and 80 mg of stearamine (C18) were added to 10 ml of N,N-dimethylformamide (DMF) and mixed at 80 °C. To a thoroughly mixed solution, 240 mg of 1-ethyl-3-(3-dimethylaminopropyl)carbodiimide [EDC] and 145 mg of N-hydroxysuccinimide (NHS) were added and stirred for one day. After the reaction, the solution was purified by dialysis for 2 days under a condition that a molecular cut-off (MWCO) against distilled water is 2,000, followed by lyophilization to prepare polyethylene glycol-TL-stearamine (PTS) (FIG. 2).

In order to measure a critical micelle concentration (CMC) of the polymeric micelles prepared from PTS, aqueous solutions containing the polymeric micelles at various concentrations were prepared. As a fluorescent probe, $6.0 \times 10^{-7}$ M of pyrene was used, the excitation wavelength was set to 336 nm in a fluorescence spectrophotometer, and the emission wavelength was observed in the range of 360 to 450 nm. As a result of the measurement, the CMC of the PTS polymeric micelles was 0.2 mg/mL.

In order to confirm the formation of polymeric micelles, after preparing a solvent in which PTS was self-assembled on a D$_2$O solvent, 1H-NMR of a D$_2$O solution of PTS was measured. PTS was completely shielded in D$_2$O as a stearic group formed a hydrophobic core in the D$_2$O solvent, and the peaks at δ 0.83 and 1.23, which are peaks caused by the alkylene group, were almost disappeared. The above results suggest that PEG formed a hydrophilic shell and the stearic group formed the hydrophobic core, resulting in formation of polymeric micelles.

**Preparation Example 3. Preparation of hydrophobic manganese oxide-micelle complex**

2 mg of hydrophobic manganese oxide having a C12 alkyl group of Preparation Example 1 was dissolved in 500 $\mu$L of anhydrous DMSO, 10 mg of PTS polymeric micelles were dissolved in 500 $\mu$L of anhydrous DMSO, and these two solutions were mixed to prepare a mixed solution having 1 mL total volume. After putting 20 mL of tertiary distilled water in a 50 mL tube, 1 mL of the mixed solution was added dropwise under probe sonication. At this time, the conditions of probe sonication were 35% amplitude in a pulse mode of 5 seconds on followed by 5 seconds off, and the sonication was performed for 5 minutes. The prepared solution was subjected to membrane dialysis (12-14 kDa MWCO) for one day to remove DMSO and residual impurities. Further, a qualitative filter paper (6-10 $\mu$m) was used to filter out particles with abnormal size once more. Finally, the filtered particles were lyophilized to obtain a micelle complex supported with solid hydrophobic manganese oxide.

**Preparation Example 2. Preparation of micelles**

Step 1: Preparation of thioketal linker (TL)

49 mmol of 3-mercaptopropionic acid was dissolved in 98 mmol of anhydrous acetone, and the resulting product was cooled while stirring constantly at 23 °C for 6 hours until crystallization. The crystallized product was filtered, rinsed with n-hexane, and then rinsed once more with cold distilled water, followed by lyophilization.

Step 2: Synthesis of thioketal linker-conjugated polyethylene glycol (PEG-TL)

254 mg of the thioketal linker (TL) prepared in Step 1 and 200 mg of methoxy-polyethylene glycol amine (PEG-AM) having a molecular weight of 2 kDa were mixed with 10 ml of N,N-dimethylformamide (DMF) and mixed at room temperature to prepare a mixed solution. Thereafter, 278 mg of 1-ethyl-3-(3-dimethylaminopropyl)carbodiimide [EDC] and 345 mg of N-hydroxysuccinimide (NHS) were added to the mixed solution prepared above, and the mixture was stirred for 16 hours. After stirring, the mixed solution was dialyzed under a condition that a molecular cut-off (MWCO) against distilled water is 1,000, so as to remove reaction by-products, followed by lyophilization. The lyophilized powder was re-dissolved in 1 ml of DMF and precipitated in cold diethyl ether 5 times. Then, the resulting product was vacuum dried to prepare thioketal linker-conjugated polyethylene glycol (PEG-TL).

Step 3: Production of polyethylene glycol-TL-stearamine (PTS)

100 mg of PEG-TL prepared in Step 2 above, 80 ml of triethylamine (TEA) and 80 mg of stearamine (C18) were added to 10 ml of N,N-dimethylformamide (DMF) and mixed at 80 °C. To a thoroughly mixed solution, 240 mg of 1-ethyl-3-(3-dimethylaminopropyl)carbodiimide [EDC] and 145 mg of N-hydroxysuccinimide (NHS) were added and stirred for one day. After the reaction, the solution was purified by dialysis for 2 days under a condition that a molecular cut-off (MWCO) against distilled water is 2,000, followed by lyophilization to prepare polyethylene glycol-TL-stearamine (PTS) (FIG. 2).

In order to measure a critical micelle concentration (CMC) of the polymeric micelles prepared from PTS, aqueous solutions containing the polymeric micelles at various concentrations were prepared. As a fluorescent probe, $6.0 \times 10^{-7}$ M of pyrene was used, the excitation wavelength was set to 336 nm in a fluorescence spectrophotometer, and the emission wavelength was observed in the range of 360 to 450 nm. As a result of the measurement, the CMC of the PTS polymeric micelles was 0.2 mg/mL.

In order to confirm the formation of polymeric micelles, after preparing a solvent in which PTS was self-assembled on a $D_2O$ solvent, 1H-NMR of a $D_2O$ solution of PTS was measured. PTS was completely shielded in $D_2O$ as a steric group formed a hydrophobic core in the $D_2O$ solvent, and the peaks at $\delta$ 0.83 and 1.23, which are peaks caused by the alkylene group, were almost disappeared. The above results suggest that PEG formed a hydrophilic shell and the steric group formed the hydrophobic core, resulting in formation of polymeric micelles.

**Preparation Example 3. Preparation of hydrophobic manganese oxide-micelle complex**

2 mg of hydrophobic manganese oxide having a C12 alkyl group of Preparation Example 1 was dissolved in 500 $\mu$L of anhydrous DMSO, 10 mg of PTS polymeric micelles were dissolved in 500 $\mu$L of anhydrous DMSO, and these two solutions were mixed to prepare a mixed solution having 1 mL total volume. After putting 20 mL of tertiary distilled water in a 50 mL tube, 1 mL of the mixed solution was added dropwise under probe sonication. At this time, the conditions of probe sonication were 35% amplitude in a pulse mode of 5 seconds on followed by 5 seconds off, and the sonication was performed for 5 minutes. The prepared solution was subjected to membrane dialysis (12-14 kDa MWCO) for one day to remove DMSO and residual impurities. Further, a qualitative filter paper (6-10 $\mu$m) was used to filter out particles with abnormal size once more. Finally, the filtered particles were lyophilized to obtain a micelle complex supported with solid hydrophobic manganese oxide.

**Experimental Example 1. Analysis of hydrophobic manganese oxide**

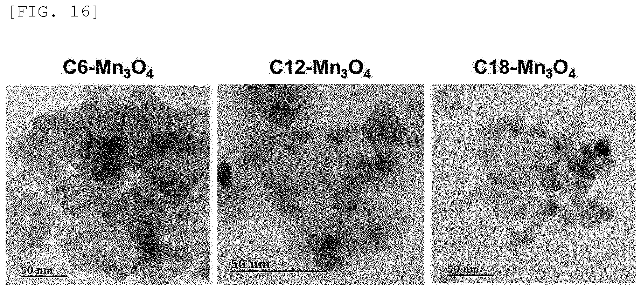

TEM image analysis of the hydrophobic manganese oxide particles prepared by the method of Preparation Example 1 was performed. As shown in FIG. 16, it can be confirmed that hydrophobic manganese oxide nanoparticles are stably formed regardless of the length of the alkyl group.

For the hydrophobic manganese oxide particles having a C12 alkyl group prepared by the method of Preparation Example 1, XPS spectrum analysis, EDS elemental analysis, and TEM image analysis were performed.

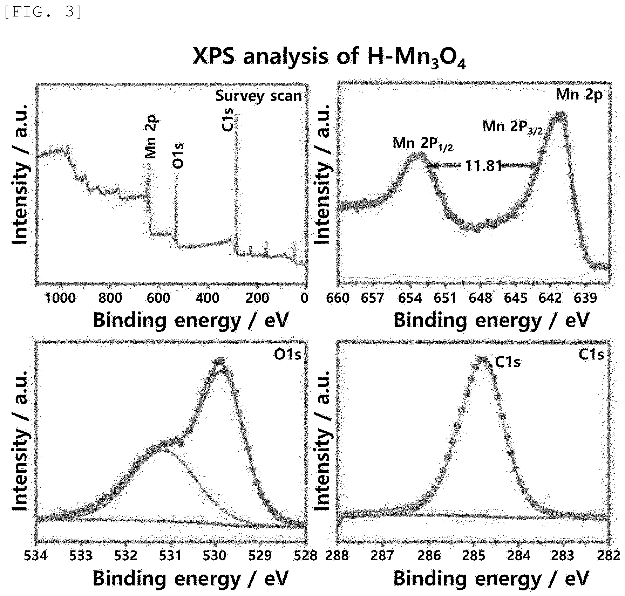

As a result of XPS spectrum analysis (FIG. 3), the XPS spectrum of Mn 2P consists of two peaks located at 641.42 eV and 653.23 eV, and these peaks were attributed to $Mn2P_{3/2}$ and $Mn2P_{1/2}$, respectively. An energy gap between these two peaks is 11.81 eV, which is not consistent with the reported literature on $Mn_3O_4$.

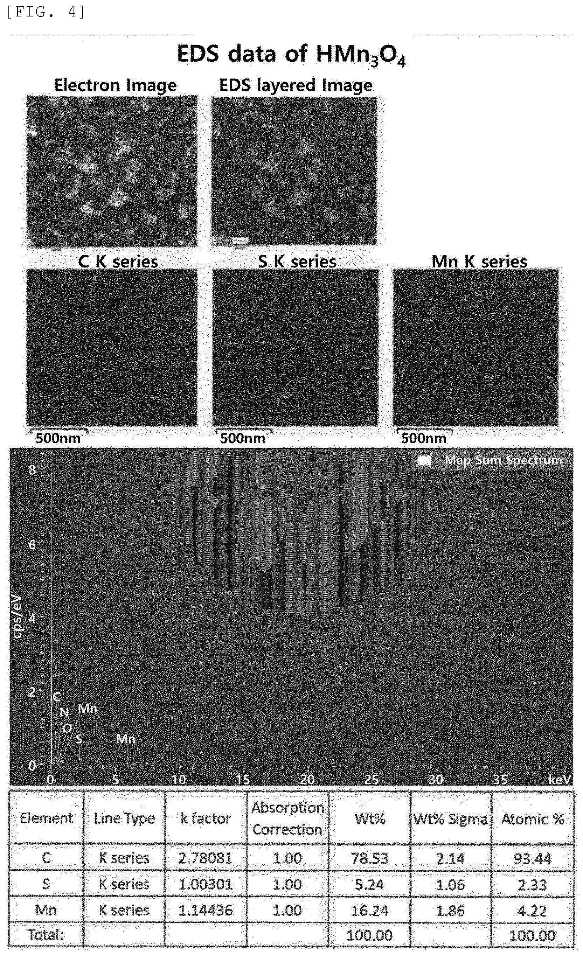

As a result of EDS elemental analysis (FIG. 4), through a comparison between the position of the hydrophobic manganese oxide nanoparticles and the position of the detected element in the electron image, it was confirmed that the particles contained Mn, and it can be seen that element S originates from C12-SH covering the surface of the hydrophobic manganese oxide.

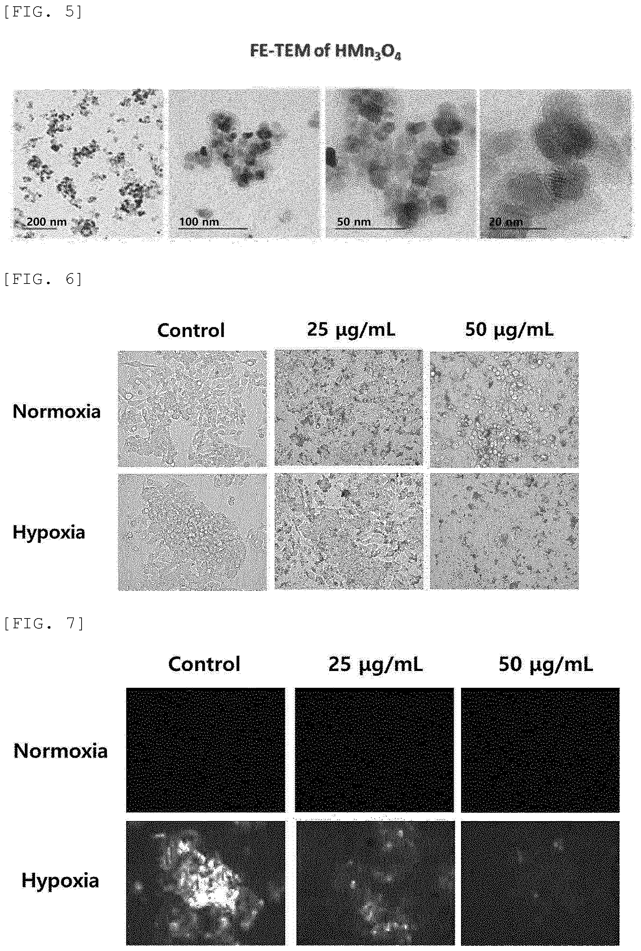

As a result of TEM image analysis (FIG. 5), it was observed that the measured size of the hydrophobic manganese oxide nanoparticles was 20 nm or less.

**Experimental Example 2. Oxygen generation effect of hydrophobic manganese oxide-micelle complex**

2-1. Hepatocellular carcinoma cells

2-1-1. Experiment method

HepG2 tumor (hepatocellular carcinoma) cells were inoculated into a 24-well plate at $1 \times 10^5$ cell/well, and an experiment was performed two days later. In the case of hypoxia environment, it entered a hypoxia chamber (incubator) for 6 hours. As a staining reagent, Hypoxia Sensing fluorescent probe, that is, Image-iT green hypoxia reagent was used.

2-1-2. Experiment result

As a result of taking bright field images of cancer cells used in the experiment (FIG. 6), it could be seen that all cancer cells used in the experiment are in a stable and healthy state regardless of hypoxia or normoxia environment.

As a result of taking green fluorescent protein (GFP) images (FIG. 7), green fluorescence (bright portion in FIG. 7) did not appear at all in the control of the normoxia environment, indicating that it was not a hypoxic environment. In the case of normoxia environment, green fluorescence was not shown regardless of whether or not the hydrophobic manganese oxide-micelle complex was treated.

In the case of the control of the hypoxia environment, very strong green fluorescence was shown (FIG. 7), indicating that it was a hypoxic environment. When the hydrophobic manganese oxide-micelle complex (25 $\mu$g/mL, 50 $\mu$g/mL) was treated in a hypoxia environment, it can be seen that the fluorescence intensity (bright portion in FIG. 7) is gradually decreased. This indicates that the hydrophobic manganese oxide-micelle complex changed the hypoxic environment into a normoxia environment by generating oxygen in the hypoxia environment.

2-2. Colorectal cancer cells

2-2-1. Experiment method

CT-26 tumor (colorectal cancer) cells were inoculated into a 24-well plate at $1 \times 10^5$ cells/well, and an experiment was performed two days later. In the case of hypoxia environment, it entered a hypoxia chamber (incubator) for 6 hours. As a staining reagent, Hypoxia Sensing fluorescent probe, that is, Image-iT green hypoxia reagent was used.

2-2-2. Experiment result

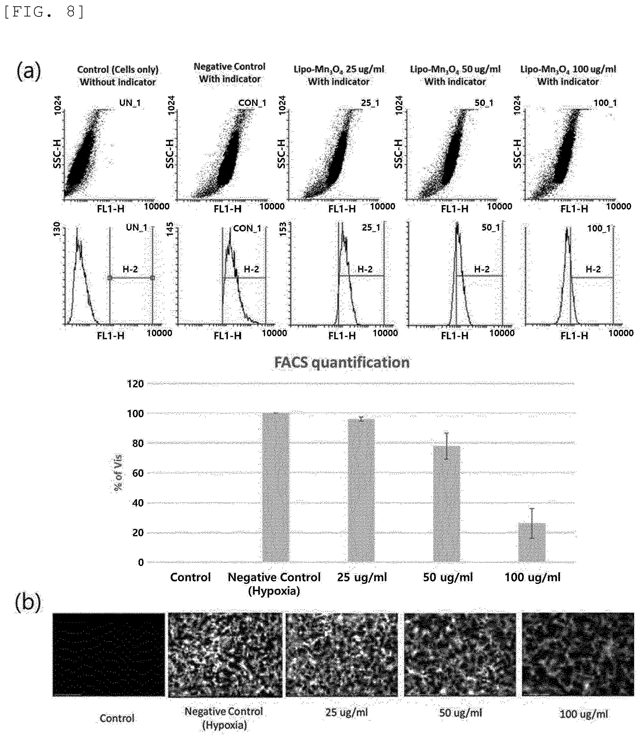

A degree of hypoxia in colorectal cancer cells was measured by a fluorescence-activated cell sorting (FACS) device using a hypoxia indicator. As a result (FIG. 8a), it can be seen that the degree of hypoxia is decreased according to the amount of the used hydrophobic manganese oxide-micelle complex. Referring to images taken with a fluorescence microscope using the same marker (FIG. 8b), it can be seen that fluorescence intensity similar to the FACS measurement result of FIG. 8a is observed.

**Experimental Example 3. Anticancer effect of the hydrophobic manganese oxide-micelle complex on hepatocellular carcinoma**

3-1. Experiment method

To confirm anticancer effects of the hydrophobic manganese oxide-micelle complex on hepatocellular carcinoma, a hepatocellular carcinoma (HepG2) animal model was used. Group settings were as follows: NC (negative control); RO (Radiation only, group with radiation therapy only) 5 animals; RL (Radiation + Low dosage sample, group with radiation therapy and intravenous injection of hydrophobic manganese oxide-micelle complex at a low concentration (1 mg [Mn]/kg)) 5 animals; and RH (Radiation + High dosage sample, group with radiation therapy and intravenous injection of hydrophobic manganese oxide-micelle complex at a high concentration (7 mg [Mn]/kg)) 5 animals.

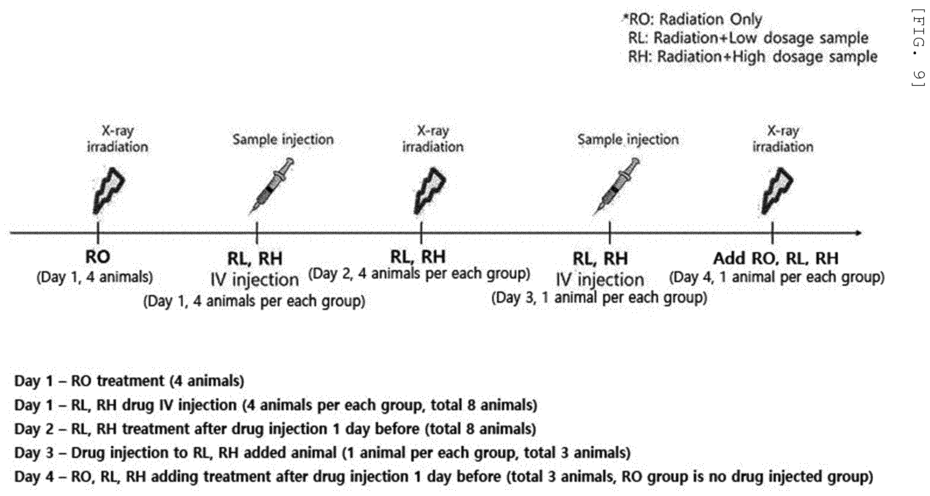

The specific experimental process is as shown in FIG. 9. On the first day, 4 rats of the RO group were treated with radiation. On the same day, 1 mg[Mn]/kg and 7 mg[Mn]/kg of the hydrophobic manganese oxide-micelle complexes were intravenously injected into 4 animals of the RL group and 4 animals of the RH group, respectively. On the following day as the 2nd day, 4 animals in the RL group and 4 animals in the RH group were treated with radiation. On the 3rd day, one animal from the RL group and one animal from the RH group were added and intravenously injected with 1 mg[Mn]/kg and 7 mg[Mn]/kg of the hydrophobic manganese oxide-micelle complexes, respectively. Further, on the following day as the 4th day, one animal of the RL group and one animal of the RH group were treated with radiation. On the 4th day, one animal of the RO group was added and treated with radiation.

3-2. Experiment result

Changes in tumor volume and body weight, and survival rate were investigated for each group, and H&E (Hematoxylin and eosin) staining and DAPI (4',6-diamidino-2-phenylindole) staining were performed.

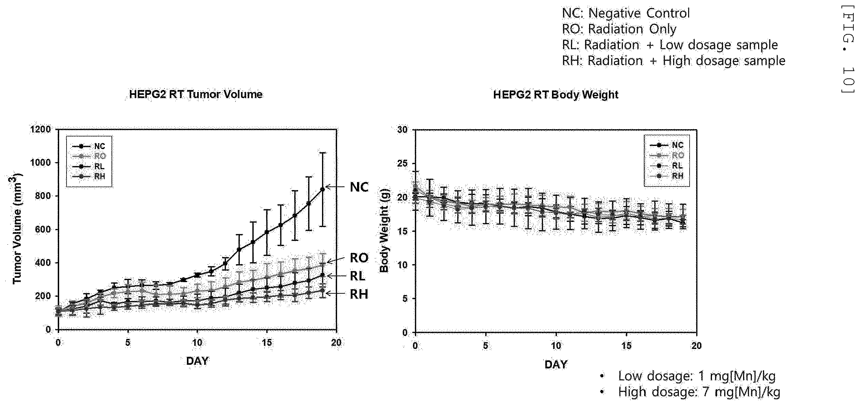

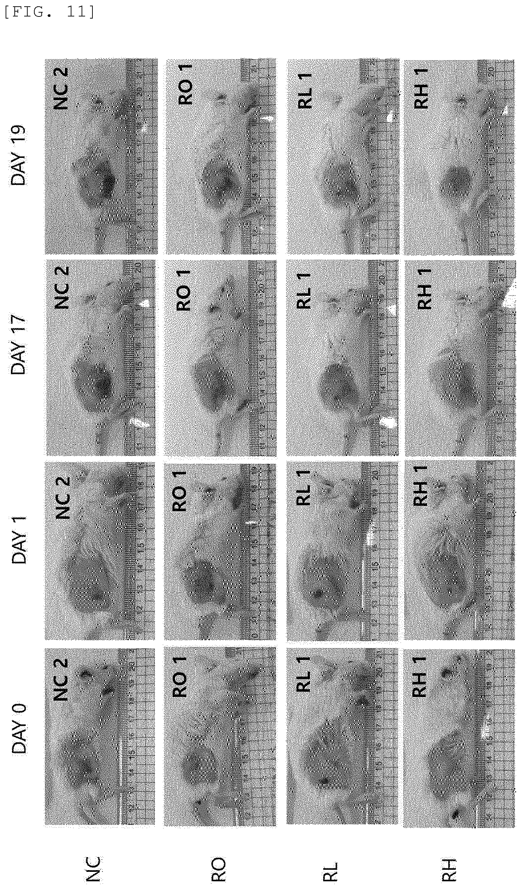

Compared to the NC (negative control), the RO, RL and RH groups all showed significantly lower increase in the tumor volume. In particular, the RH group showed the lowest increase in the tumor volume, demonstrating that it attained best tumor volume reduction (FIG10a). On the other hand, there was no significant difference in the body weight by group (FIG. 10b). As shown in FIG. 11, it can also be confirmed visually from actual animal photographs that the tumor volume of the RH group is significantly reduced.

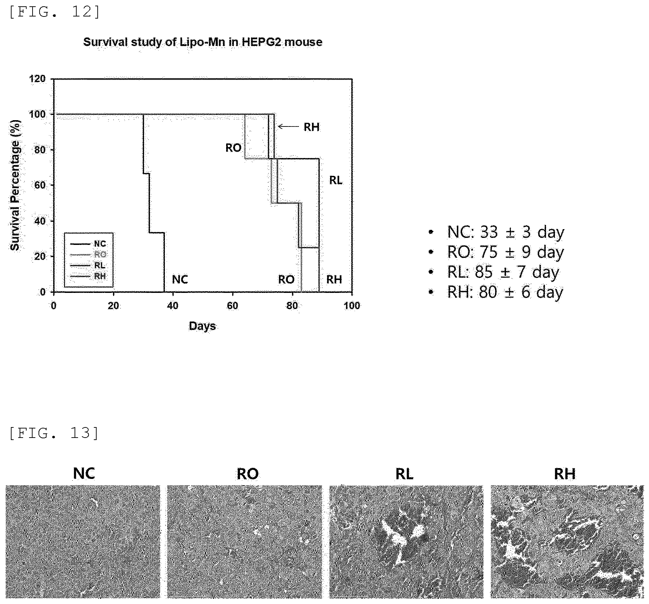

As a result of confirming the survival rate (FIG. 12), it can be seen that the RO group (75 $\pm$ 9 days), RL group (85 $\pm$ 7 days) and RH group (80 $\pm$ 6 days) exhibit all high survival rate, compared to NC (33 $\pm$ 3 days). In particular, it can be seen that the RL group and the RH group survived the longest period of time.

As a result of H&E staining (FIG. 13), it can be confirmed that a degree of tumor necrosis is severe in the RL and RH groups compared to the NC and RO group. Especially, it can be seen that the tumor necrosis most actively proceeded in the RH group.

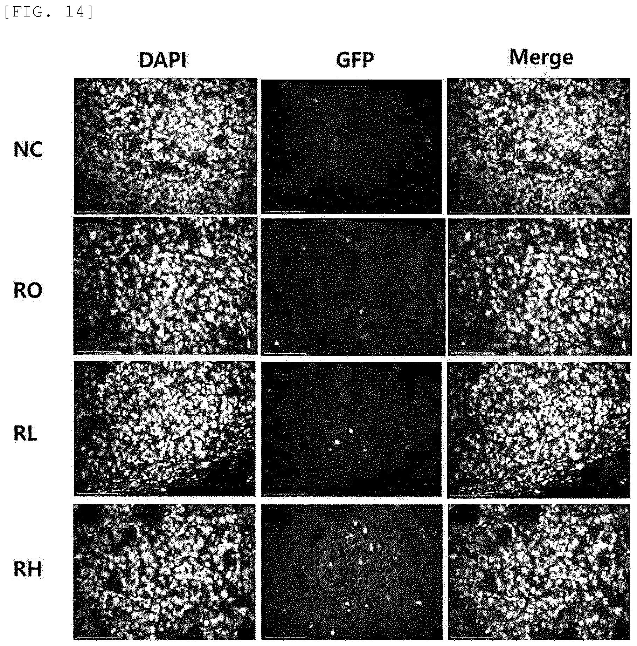

As a result of DAPI staining (FIG. 14), it can be confirmed that a degree of cell apoptosis displayed in green (FIG. 14, bright part of the GFP image) is severe in the RL and RH groups compared to the NC and RO group. Especially, it can be seen that cell apoptosis most actively proceeded in the RH group.

**Claims**

1. A composition for enhancing radiation therapy, comprising a complex including:

   micelles formed of amphiphilic molecules in which a hydrophilic portion and a hydrophobic portion are bound by a reactive oxygen species (ROS) cleavable linker; and
   manganese oxide particles which are supported on cores of the micelles and hydrophobically modified by binding a hydrophobic substituent to the surface thereof.

2. The composition for enhancing radiation therapy according to claim 1, wherein the active oxygen-cleavable linker is a thioketal linker.

3. The composition for enhancing radiation therapy according to claim 1, wherein the hydrophobic portion of the amphiphilic molecule includes a C12 to C22 aliphatic hydrocarbon group.

4. The composition for enhancing radiation therapy according to claim 3, wherein the aliphatic hydrocarbon group is any one of a C12 to C22 alkyl group and a C12 to C22 alkenyl group.

5. The composition for enhancing radiation therapy according to claim 1, wherein the hydrophilic portion of the amphiphilic molecule includes a water-soluble polymer.

6. The composition for enhancing radiation therapy according to claim 5, wherein the water-soluble polymer is polyethylene glycol, acrylic acid polymer, methacrylic acid polymer, polyamidoamine, poly(2-hydroxypropyl methacrylamide) polymer, water-soluble polypeptide, water-soluble polysaccharide or polyglycerol.

7. The composition for enhancing radiation therapy according to claim 1, wherein the hydrophobic substituent is a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a halogen group, a C1 to C30 ester group or a halogen-containing group.

8. The composition for enhancing radiation therapy according to claim 1, wherein the hydrophobic substituent is a substituted or unsubstituted C1 to C30 alkyl group.

9. The composition for enhancing radiation therapy according to claim 1, wherein the manganese oxide is any one of $MnO$, $Mn_2O_3$, $MnO_2$, $Mn_3O_4$ and $Mn_2O_3$.

10. The composition for enhancing radiation therapy according to claim 1, wherein the manganese oxide particles have a particle diameter of 10 nm to 30 nm.

11. The composition for enhancing radiation therapy according to any one of claims 1 to 10, further comprising a drug carried on the micelles.

12. A composition for enhancing photodynamic therapy, comprising a complex including:

micelles formed of amphiphilic molecules in which a hydrophilic portion and a hydrophobic portion are bound by a reactive oxygen species (ROS) cleavable linker; and
manganese oxide particles which are supported on cores of the micelles and hydrophobically modified by binding a hydrophobic substituent to the surface thereof.

13. The composition for enhancing photodynamic therapy according to claim 12, wherein the active oxygen-cleavable linker is a thioketal linker.

14. The composition for enhancing photodynamic therapy according to claim 12, wherein the hydrophobic portion of the amphiphilic molecule includes a C12 to C22 aliphatic hydrocarbon group.

15. The composition for enhancing photodynamic therapy according to claim 14, wherein the aliphatic hydrocarbon group is any one of a C12 to C22 alkyl group and a C12 to C22 alkenyl group.

16. The composition for enhancing photodynamic therapy according to claim 12, wherein the hydrophilic portion of the amphiphilic molecule includes a water-soluble polymer.

17. The composition for enhancing photodynamic therapy according to claim 16, wherein the water-soluble polymer is polyethylene glycol, acrylic acid polymer, methacrylic acid polymer, polyamidoamine, poly(2-hydroxypropyl methacrylamide) polymer, water-soluble polypeptide, water-soluble polysaccharide or polyglycerol.

18. The composition for enhancing photodynamic therapy according to claim 12, wherein the hydrophobic substituent is a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a halogen group, a C1 to C30 ester group or a halogen-containing group.

19. The composition for enhancing photodynamic therapy according to claim 12, wherein the hydrophobic substituent is a substituted or unsubstituted C1 to C30 alkyl group.

20. The composition for enhancing photodynamic therapy according to claim 12, wherein the manganese oxide is any one of MnO, $Mn_2O_3$, $MnO_2$, $Mn_3O_4$ and $Mn_2O_5$.

21. The composition for enhancing photodynamic therapy according to claim 12, wherein the manganese oxide particles have a particle diameter of 10 nm to 30 nm.

22. The composition for enhancing photodynamic therapy according to any one of claims 12 to 21, further comprising a drug carried on the micelles.

23. A composition for enhancing ultrasound dynamic therapy, comprising a complex including:

micelles formed of amphiphilic molecules in which a hydrophilic portion and a hydrophobic portion are bound by a reactive oxygen species (ROS) cleavable linker; and
manganese oxide particles which are supported on cores of the micelles and hydrophobically modified by binding a hydrophobic substituent to the surface thereof.

24. The composition for enhancing ultrasound dynamic therapy according to claim 23, wherein the active oxygen-cleavable linker is a thioketal linker.

25. The composition for enhancing ultrasound dynamic therapy according to claim 23, wherein the hydrophobic portion of the amphiphilic molecule includes a C12 to C22 aliphatic hydrocarbon group.

26. The composition for enhancing ultrasound dynamic therapy according to claim 25, wherein the aliphatic hydrocarbon group is any one of a C12 to C22 alkyl group and a C12 to C22 alkenyl group.

27. The composition for enhancing ultrasound dynamic therapy according to claim 23, wherein the hydrophilic portion of the amphiphilic molecule includes a water-soluble polymer.

28. The composition for enhancing ultrasound dynamic therapy according to claim 27, wherein the water-soluble polymer is polyethylene glycol, acrylic acid polymer, methacrylic acid polymer, polyamidoamine, poly(2-hydroxypropyl methacrylamide) polymer, water-soluble polypeptide, water-soluble polysaccharide or polyglycerol.

29. The composition for enhancing ultrasound dynamic therapy according to claim 23, wherein the hydrophobic substituent is a substituted or unsubstituted C1 to C30 alkyl group, a substituted or unsubstituted C3 to C30 cycloalkyl group, a substituted or unsubstituted C6 to C30 aryl group, a substituted or unsubstituted C2 to C30 heteroaryl group, a halogen group, a C1 to C30 ester group or a halogen-containing group.

30. The composition for enhancing ultrasound dynamic therapy according to claim 23, wherein the hydrophobic substituent is a substituted or unsubstituted C1 to C30 alkyl group.

31. The composition for enhancing ultrasound dynamic therapy according to claim 23, wherein the manganese oxide is any one of MnO, $Mn_2O_3$, $MnO_2$, $Mn_3O_4$ and $Mn_2O_5$.

32. The composition for enhancing ultrasound dynamic therapy according to claim 23, wherein the manganese oxide particles have a particle diameter of 10 nm to 30 nm.

33. The composition for enhancing ultrasound dynamic therapy according to any one of claims 23 to 32, further comprising a drug carried on the micelles.

[FIG. 1]

Schematic view of Hydrophobic Mn3O4 nanoparticles

[FIG. 2]

[FIG. 3]

XPS analysis of H-Mn₃O₄

[FIG. 4]

# EDS data of HMn₃O₄

### Electron Image

### EDS layered Image

### C K series

### S K series

### Mn K series

500nm    500nm    500nm

| Element | Line Type | k factor | Absorption Correction | Wt% | Wt% Sigma | Atomic % |
|---------|-----------|----------|----------------------|------|-----------|----------|
| C | K series | 2.78081 | 1.00 | 78.53 | 2.14 | 93.44 |
| S | K series | 1.00301 | 1.00 | 5.24 | 1.06 | 2.33 |
| Mn | K series | 1.14436 | 1.00 | 16.24 | 1.86 | 4.22 |
| Total: | | | | 100.00 | | 100.00 |

[FIG. 5]

**FE-TEM of HMn$_3$O$_4$**

[FIG. 6]

[FIG. 7]

[FIG. 8]

(a) Control (Cells only) Without indicator / Negative Control With indicator / Lipo-Mn₃O₄ 25 ug/ml With indicator / Lipo-Mn₃O₄ 50 ug/ml With indicator / Lipo-Mn₃O₄ 100 ug/ml With indicator

FACS quantification

(b) Control / Negative Control (Hypoxia) / 25 ug/ml / 50 ug/ml / 100 ug/ml

*RO: Radiation Only
RL: Radiation+Low dosage sample
RH: Radiation+High dosage sample

X-ray irradiation — RO (Day 1, 4 animals)

Sample injection — RL, RH IV injection (Day 1, 4 animals per each group)

X-ray irradiation — RL, RH (Day 2, 4 animals per each group)

Sample injection — RL, RH IV injection (Day 3, 1 animal per each group)

X-ray irradiation — Add RO, RL, RH (Day 4, 1 animal per each group)

Day 1 – RO treatment (4 animals)
Day 1 – RL, RH drug IV injection (4 animals per each group, total 8 animals)
Day 2 – RL, RH treatment after drug injection 1 day before (total 8 animals)
Day 3 – Drug injection to RL, RH added animal (1 animal per each group, total 3 animals)
Day 4 – RO, RL, RH adding treatment after drug injection 1 day before (total 3 animals, RO group is no drug injected group)

[FIG. 9]

[FIG. 10]

NC: Negative Control
RO: Radiation Only
RL: Radiation + Low dosage sample
RH: Radiation + High dosage sample

EP 4 371 557 A1

HEPG2 RT Tumor Volume

HEPG2 RT Body Weight

• Low dosage: 1 mg[Mn]/kg
• High dosage: 7 mg[Mn]/kg

[FIG. 11]

[FIG. 12]

**Survival study of Lipo-Mn in HEPG2 mouse**

- NC: 33 ± 3 day
- RO: 75 ± 9 day
- RL: 85 ± 7 day
- RH: 80 ± 6 day

[FIG. 13]

[FIG. 14]

NaOH mediated reduction to form Mn₃O₄ followed by stabilization using thiols of C6, C12 or C18

[FIG. 16]

C6-Mn$_3$O$_4$      C12-Mn$_3$O$_4$      C18-Mn$_3$O$_4$

<table>
<tr><td colspan="2" align="center">**INTERNATIONAL SEARCH REPORT**</td><td colspan="2">International application No.<br><br>**PCT/KR2022/009876**</td></tr>
<tr><td colspan="4">**A.    CLASSIFICATION OF SUBJECT MATTER**<br>**A61K 9/127**(2006.01)i; **A61K 47/69**(2017.01)i; **A61K 33/32**(2006.01)i; **A61K 45/06**(2006.01)i; **A61P 35/00**(2006.01)i<br><br>According to International Patent Classification (IPC) or to both national classification and IPC</td></tr>
<tr><td colspan="4">**B.    FIELDS SEARCHED**</td></tr>
<tr><td colspan="4">Minimum documentation searched (classification system followed by classification symbols)<br>A61K 9/127(2006.01); A61K 31/704(2006.01); A61K 47/69(2017.01); A61K 9/107(2006.01)</td></tr>
<tr><td colspan="4">Documentation searched other than minimum documentation to the extent that such documents are included in the fields searched<br>Korean utility models and applications for utility models: IPC as above<br>Japanese utility models and applications for utility models: IPC as above</td></tr>
<tr><td colspan="4">Electronic data base consulted during the international search (name of data base and, where practicable, search terms used)<br>eKOMPASS (KIPO internal) & keywords: 산화망간(Manganese oxide, MnO), 마이셀(micelle), 티오케탈 링커(thioketal linker), 활성산소-분해성(ROS-cleavable), 방사선 치료(radiation therapy), 광역학 치료(photodynamic therapy)</td></tr>
<tr><td colspan="4">**C.    DOCUMENTS CONSIDERED TO BE RELEVANT**</td></tr>
<tr><td>Category*</td><td colspan="2">Citation of document, with indication, where appropriate, of the relevant passages</td><td>Relevant to claim No.</td></tr>
<tr><td>A</td><td colspan="2">HOWELL, M. et al. Manganese-loaded lipid-micellar theranostics for simultaneous drug and gene delivery to lungs. Journal of Controlled Release. 2013, vol. 167, pp. 210-218 (online publication date: 06 February 2013).<br>See abstract; pages 211-213; and figure 1.</td><td>1-33</td></tr>
<tr><td>A</td><td colspan="2">UTHAMAN, S. et al. Tumor microenvironment-responsive nanoparticles for cancer theragnostic applications. Biomaterials Research. 2018, vol. 22, no. 3, pp. 172-182 (online publication date:23 August 2018).<br>See page 179, right column.</td><td>1-33</td></tr>
<tr><td>A</td><td colspan="2">SUN, C. et al. A ROS-responsive polymeric micelle with a π-conjugated thioketal moiety for enhanced drug loading and efficient drug delivery. Organic & Biomolecular Chemistry. 09 October 2017 (publication date), vol. 15, pp. 9176-9185.<br>See abstract; and scheme 1.</td><td>1-33</td></tr>
<tr><td>A</td><td colspan="2">XIANG, Y. et al. Biocompatible and pH-sensitive MnO-loaded carbonaceous nanospheres (MnO@CNSs): A theranostic agent for magnetic resonance imaging-guided photothermal therapy. Carbon. 2018, vol. 136, pp. 113-124 (online publication date: 23 April 2018).<br>See abstract.</td><td>1-33</td></tr>
<tr><td colspan="4">☑ Further documents are listed in the continuation of Box C.        ☑ See patent family annex.</td></tr>
<tr><td colspan="2">*    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed</td><td colspan="2">"T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family</td></tr>
<tr><td colspan="2">Date of the actual completion of the international search<br><br>**18 October 2022**</td><td colspan="2">Date of mailing of the international search report<br><br>**18 October 2022**</td></tr>
<tr><td colspan="2">Name and mailing address of the ISA/KR<br><br>**Korean Intellectual Property Office**<br>**Government Complex-Daejeon Building 4, 189 Cheongsa-**<br>**ro, Seo-gu, Daejeon 35208**<br><br>Facsimile No. **+82-42-481-8578**</td><td colspan="2">Authorized officer<br><br><br><br><br><br><br><br>Telephone No.</td></tr>
</table>

Form PCT/ISA/210 (second sheet) (July 2022)

**INTERNATIONAL SEARCH REPORT**

| International application No. |
|---|
| **PCT/KR2022/009876** |

**C.      DOCUMENTS CONSIDERED TO BE RELEVANT**

| Category* | Citation of document, with indication, where appropriate, of the relevant passages | Relevant to claim No. |
|---|---|---|
| A | CN 108578364 A (STOMATOLOGICAL HOSPITAL, TIANJIN MEDICAL UNIVERSITY) 28 September 2018 (2018-09-28)<br>    See claims 1 and 2. | 1-33 |
| PX | KR 10-2395946 B1 (DR. CURE) 10 May 2022 (2022-05-10)<br>    See claims 1-16.<br>    'This document is a published earlier application that serves as a basis for claiming priority of the present international application.' | 1-33 |

Form PCT/ISA/210 (second sheet) (July 2022)

**INTERNATIONAL SEARCH REPORT**
Information on patent family members

International application No.

**PCT/KR2022/009876**

| Patent document cited in search report | | | Publication date (day/month/year) | Patent family member(s) | | | Publication date (day/month/year) |
|---|---|---|---|---|---|---|---|
| CN | 108578364 | A | 28 September 2018 | None | | | |
| KR | 10-2395946 | B1 | 10 May 2022 | KR 10-2022-0008243 | | A | 20 January 2022 |

Form PCT/ISA/210 (patent family annex) (July 2022)